# EUROPÄISCHE PATENTSCHRIFT

(11) **EP 1 194 127 B1**
(45) Veröffentlichungstag und Bekanntmachung des Hinweises auf die Patenterteilung: **10.11.2004**
(21) Anmeldenummer: 00954496.6
(22) Anmeldetag: 13.07.2000
(51) Int. Cl.: A61K 9/16, B01J 3/00

(54) **VERFAHREN ZUR HERSTELLUNG PULVERFÖRMIGER PARTIKELREDUZIERTER FORMULIERUNGEN MIT HILFE VERDICHTETER GASE**
METHOD FOR PRODUCING POWDERY PARTICLE-REDUCED FORMULATIONS WITH THE AID OF COMPRESSED GASES
PROCEDE DE PREPARATION DE FORMULATIONS PULVERULENTES A TAUX PARTICULAIRE REDUIT A L'AIDE DE GAZ COMPRIMES

(30) Priorität: 13.07.1999 DE 19932648; 14.12.1999 DE 19960167
(43) Veröffentlichungstag der Anmeldung: 10.04.2002
(73) Patentinhaber: Degussa AG, 83308 Trostberg (DE)
(72) Erfinder: HEIDLAS, Jürgen, 83308 Trostberg (DE); OBER, Martin, 83352 Altenmarkt (DE); WIESMÜLLER, Johann, 84518 Garching (DE)
(74) Vertreter: Weiss, Wolfgang, Dipl.-Chem. Dr.
(86) Internationale Anmeldenummer: PCT/EP2000/006709
(87) Internationale Veröffentlichungsnummer: WO 2001/003671

(56) Entgegenhaltungen:
- EP-A- 0 542 314
- EP-A- 0 677 332
- WO-A-94/18264
- WO-A-99/52504
- DE-A- 4 041 563

## Beschreibung

Die vorliegende Erfindung beschreibt ein Verfahren zur Herstellung pulverförmiger partikelreduzierter Formulierungen mit Hilfe verdichteter Gase.

Eine beträchtliche Anzahl von Substanzen wie Wirkstoffe, Lacke und Additive besitzt oft eine nur geringe Löslichkeit in Wasser sowie in vielen organischen Lösemitteln. Die schlechte Löslichkeit von speziell Pharmawirkstoffen stellt sowohl eine Herausforderung an die pharmazeutischen Technologen dar, die versuchen, mit verschiedenen Formulierungsstrategien, wie einer Reduktion der Partikelgröße und/oder der Einbettung in geeignete Additive eine Löslichkeitsverbesserung zu erzielen (Voigt, R., "Pharmazeutische Technologie" 7. Auflage, Berlin: Ullstein Mosby, 1995), als auch für die Wirkstoffsynthetiker, die versuchen, durch strukturelle Veränderungen an den Wirkstoffmolekülen selbst deren Löslichkeit zu verbessern, ohne dabei gleichzeitig die Wirkprinzipien negativ zu beeinflussen (Wermuth, C.G., "Preparation of Water-Soluble Compounds by Covalent Attachment of Solubilizing Moieties" in The Practice of Medicinal Chemistry, New York: Academic Press, pp. 755 - 776, 1996).

Bei den Verfahrensweisen der pharmazeutischen Technologie stehen klassische Strategien zur Reduzierung der Partikelgröße und/oder zur Einbettung der Wirkstoffmoleküle in eine löslichkeits- bzw. absorptionsfördernde Matrix im Vordergrund (Muranishi, S., Crit. Rev. Ther. Drug Carrier Syst. 7, 1 - 33 (1990)). Es werden auch Lipidvesikel, insbesondere Liposomen als Wirkstoff-Carrier vorgeschlagen (Lasic, D. D., J. Controlled Drug Release 48, 203 - 222 (1997)), die eine gezielte Verbesserung der biologischen Verfügbarkeit der Wirkstoffe nach Applikation bewirken können.

Viele technologische Ansätze stoßen jedoch an ihre Grenzen, wenn für einen schlecht löslichen Wirkstoff durch eine Verringerung seiner Partikelgröße eine noch akzeptable biologische Verfügbarkeit nach der Applikation erreicht werden soll. Dazu stehen zwar eine Reihe klassischer Mahltechniken zur Verfügung, wie z.B. mit Hilfe von Fliehkraftmühlen, Schlagstiftmühlen, Luftstrahlmühlen, Kugelmühlen etc. (Voigt, R., "Pharmazeutische Technologie" 7. Auflage, pp. 40, Berlin: Ullstein Mosby, 1995). Diese Techniken sind aber oftmals zur Umsetzung minimal erreichbarer Partikelgrößen und akzeptabler Korngrößenverteilungen ungeeignet.

Neuere Technologien haben daher versucht, zur Herstellung mikro- bzw. nanoskaliger Wirkstoffpartikel die Eigenschaften verdichteter bzw. überkritischer Gase auszunutzen (Howdle, S. et al. Proc. Int. Symp. Controlled Release Bioact. Mater. 25, 972 (1998)), wobei sich folgende Verfahren durchgesetzt haben:
1. Das sogenannte RESS-Verfahren, bei dem der Wirkstoff im überkritischen Gas gelöst und anschließend über eine feine Düse entspannt und dabei "atomisiert" wird,
2. das sogenannte GASR-Verfahren, bei dem der gelöste Wirkstoff durch das Gas und unter Druck aus einer Lösung ausgefällt wird, und schließlich
3. das PGSS-Verfahren, bei dem u. a. Copräzipitate zwischen dem jeweiligen Wirkstoff und einem Träger, wie z.B. einem Polymer, aus einer mit Gas gesättigten Lösung versprüht werden.

Die deutschen Offenlegungsschriften DE-A-197 13096, DE-A-197 58 157 sowie DE-A-198 29 396 bspw. beschreiben den homogenen Eintrag von Wirksubstanzen mit unterschiedlichen Löslichkeitseigenschaften in verschiedene Emulgatormatrices aus Lösungen, wobei mit Hilfe komprimierter Gase ein Lösemittelwechsel in einem Kolonnensystem erreicht wird, der zu homogenen Formulierungen ursprünglich schlecht löslicher Wirksubstanzen führt.

Diese neueren Verfahren zur Partikelherstellung mit Hilfe verdichteter bzw. überkritischer Gase können jedoch nur dann eingesetzt werden, wenn die Wirkstoffe entweder zumindest teilweise im verdichteten Gas selbst oder in einem Lösemittel vollständig in Lösung gebracht werden können. Leider ist dies bei vielen Wirkstoffen, Überzugsmittein wie z. B. Farblacken, aber auch Baustoffchemikalien, wie insbesondere Betonzusatzmitteln, jedoch nicht oder nur in unbedeutendem Ausmaß der Fall, so daß diese Verfahrensweisen entweder nicht oder nur unwirtschaftlich in die Praxis umgesetzt werden können.

Aufgabe der vorliegenden Erfindung war es somit, ein Verfahren zur Herstellung pulverförmiger partikelreduzierter Fomulierungen mit Hilfe verdichteter Gase bereitzustellen, mit dem schwer- oder nichtlösliche Verbindungen so formuliert werden können, daß allgemein eine signifikante Verbesserung der Anwendbarkeit und bei bioaktiven Substanzen insbesondere eine bessere biologische Verfügbarkeit erzielt-wird.

"Partikelreduziert" bedeutet im Sinne der vorliegenden Erfindung, daß der Anteil an Partikeln mit einem Durchmesser > 50 µm gegenüber dem Ausgangsmaterial um > 90 % verringert ist.

"Schwerlöslich" bedeutet im Sinne der vorliegenden Erfindung, dass bei Temperaturen zwischen 15 und 25°C, insbesondere bei etwa 20°C, für einen Massenteil Substanz mindestens 100 Volumenteile und vorzugsweise zwischen 100 und 1000 Volumenteile Lösemittel benötigt werden (siehe auch Europäisches Arzneibuch, 3. Ausgabe 1997, 1.3 Monographien, S.3).

Gelöst wurde diese Aufgabenstellung durch ein Verfahren, bei dem man
a) die zu formulierende feste Verbindung in Gegenwart von verdichtetem Gas gemeinsam mit 10 bis 99 Gew.-% eines Trägermaterials bezogen auf das Gesamtgewicht der partikelreduzierten Formulierung aus Verbindung und Trägermaterial bei erhöhten Verfahrensdrücken homogen vermahlt, wobei das Trägermaterial im verdichteten Gas im Wesentlichen löslich ist, sowie
b) anschließend das verdichtete Gas durch Druckerniedrigung vorzugsweise auf Atmosphärendruck entspannt und vom Homogenat abtrennt sowie die pulverförmige partikelreduzierte Formulierung aus dem Homogenat gewinnt.

Überraschend konnte festgestellt werden, daß durch das neue Verfahren nicht nur pulverförmige partikelreduzierte Formulierungen, sondern Formulierungen erhalten werden, die völlig homogen sind, d.h. Formulierungen, die die zu formulierende Verbindung, also den eigentlichen Wirkstoff, quantitativ enthalten und die darüber hinaus keine lichtmikroskopisch feststellbaren Partikel dieses Wirkstoffes mehr beinhalten. Dies war in dieser Deutlichkeit nicht zu erwarten.

Als zu formulierende feste Verbindungen werden im Sinne der Erfindung schwer- oder nichtlösliche Verbindungen verstanden, insbesondere Verbindungen, die in Abwesenheit eines Trägermaterials in einem verdichteten Gas schwer- oder nichtlöslich sind. Dabei handelt es sich bevorzugt um bioaktive Verbindungen und insbesondere um pharmazeutische und kosmetische Wirkstoffe; aber auch andere schwerlösliche Stoffe, deren Löslichkeit bzw. biologische Verfügbarkeit verbessert werden soll, können eingesetzt werden. Pharmazeutische Wirkstoffe sind definitionsgemäß Substanzen, die in der medizinischen Therapie und Diagnostik geeignet sind. Beim vorliegenden Verfahren werden bevorzugt pharmazeutische Verbindungen eingesetzt, die allgemein topisch, transdermal, peroral, parenteral sowie inhalativ, intravenös, intramuskulär, subkutan, intraperitonal bzw. intranasal verabreicht werden können. Unter kosmetischen Wirkstoffen werden in der Regel Substanzen verstanden, die auf die Haut aufgebracht werden.

Bevorzugte zu formulierende feste Verbindungen können im Sinn der vorliegenden Erfindung aber auch schwer- oder nichtlösliche Agrochemikalien sein, wie insbesondere Biozide (Herbizide, Fungizide, Insektizide) oder Wirkstoffe, die als sog. Pflanzenwachstumsregulatoren (PGR) verwendet werden.

Auch Farblacke bzw. deren Inhaltsstoffe mit schlechter Löslichkeit sowie bestimmte Betonzusatzmittel, also Baustoffchemikalien allgemein fallen unter die Definition der festen Verbindung gemäß Erfindung.

Substanzen oder Substanzmischungen, in die die zu formulierende feste Verbindung eingebettet werden kann, sind typische Trägermaterialien gemäß Erfindung. Dabei kann der Anteil an Trägermaterial in weiten Bereichen variieren, wobei der Anteil aber im Wesentlichen durch die Fließfähigkeit der Formulierung determiniert wird.

Zu bevorzugen sind Anteile des Trägermaterials, die in der Formulierung zwischen 50 und 90 Gew.-% betragen. Dabei sind synthetische und polymere Träger für das vorgeschlagene Herstellungsverfahren besonders geeignet, insbesondere Polyethylenoxid-Block-Copolymere ("Poloxamere"), Polyethylenglykole, Siliconderivate wie methyl- oder phenylsubstituierter Polysiloxane mit unterschiedlichem Vernetzungsgrad oder Gelatine und deren Derivate. Besonders bevorzugt werden Substanzen mit oberflächenaktiver Wirkung, wie natürliche und synthetisch hergestellte Phospholipide, vor allem Glycerophospholipide ("Lecithine"), Partialglyceride wie Mono- und/oder Diglyceride oder Kohlenhydrate und Kohlenhydratderivate mit oberflächenaktiver Wirkung, wie z.B. Alkylpolyglycoside, Zuckerester oder Sorbitanfettsäureester, sowie deren Mischungen eingesetzt. Als wesentliche Voraussetzung zum Einsatz der Trägermaterialien ist im Sinne der Erfindung nur zu definieren, daß diese im jeweiligen verdichteten Gas entweder löslich sind oder sich ein Teil des Gases im Trägermaterial selbst löst. Letzteres äußert sich meist nur durch das Quellen des Trägermaterials unter den entsprechenden Zustandsbedingungen des erfindungsgemäßen Herstellungsverfahrens. Gut geeignet sind als Trägermaterialien pharmazeutisch akzeptable Substanzen oder deren Mischungen, die bereits als Absorptionsverbesserer speziell in der Galenik beschrieben sind.

Das vorliegende Verfahren sieht vorzugsweise vor, als verdichtete Gase Propan oder Kohlendioxid oder Dimethylether (DME), aber auch beliebige Mischungen aus Propan und Dimethylether heranzuziehen, wobei auch Gasmischungen aus Propan und/oder DME mit bis zu 90 Gew.-% bezogen auf die Gasmischung Butan oder Kohlendioxid eingesetzt werden können.

Die Vermahlung der zu formulierenden festen Verbindung mit dem Trägermaterial in Anwesenheit von verdichteten Gasen, wie im erfindungsgemäßen Verfahren vorgesehen, kann grundsätzlich mit allen geeigneten Mahltechniken, die nach dem Stand der Technik bekannt sind, durchgeführt werden (Voigt, R., "Pharmazeutische Technologie" 7. Auflage, pp. 40, Berlin: Ullstein Mosby, 1995). Als besonders geeignet hat sich jedoch die Vermahlung in einem Rührautoklaven mit mechanischer Mahleinrichtung z.B. nach dem Prinzip einer Kugelmühle bewährt, wobei die Vermahlung einfach in einem hochtourig laufenden Rührautoklaven in Gegenwart von Mahlkugein durchgeführt werden kann. Menge, Art und Beschaffenheit der Mahlkugeln sowie die Geschwindigkeit und Dauer des Mahlvorganges richten sich dabei jeweils ganz nach den Eigenschaften der zu vermahlenden festen Verbindung.

Durch den Mahlvorgang in der konzentrierten Matrix der Trägermaterialien unter Druck werden die partikulären, wie insbesondere kristallinen Strukturen der zu formulierenden festen Verbindung weitgehend vollständig aufgelöst und homogen in die Matrix eingebettet, was bspw. über lichtmikroskopische Begutachtungen des Mahlergebnisses gut nachvollzogen werden kann.

Für spezielle Verfahrensvarianten haben sich in Schritt a) für das homogene Vermahlen Rührgeschwindigkeiten geeignet erwiesen, die zwischen 500 und 4000 U/min. und insbesondere zwischen 1000 und 2000 U/min. liegen. Vermahlzeiten zwischen 1 und 2 Stunden sind besonders günstig; sie können aber auch durchaus zwischen 0,5 und 3 Stunden betragen.

Die Zustandsbedingungen werden insgesamt so gewählt, daß das Trägermaterial eine homogene Phase bilden kann, z.B. das verdichtete Gas mindestens teilweise im Trägermaterial gelöst werden kann, d.h., daß unter Umständen das Trägermaterial auch nur im gequollenen Zustand vorliegt.

Geeignete Verfahrensdrücke liegen vorzugsweise zwischen 5 und 500 bar und besonders bevorzugt zwischen 40 und 120 bar; die Verfahrenstemperaturen zur Vermahlung im verdichteten Gas liegen vorzugsweise zwischen 10 und 200°C und besonders bevorzugt zwischen 50 und 120°C, um eine thermische Schädigung der Komponenten auszuschließen.

Bei bevorzugten Verfahrensvarianten werden Bedingungen gewählt, bei denen das Trägermaterial, insbesondere die oberflächenaktive Substanz, im verdichteten Gas als Schmelze, d.h. in einem 2-Phasensystem, vorliegt: In der leichteren Oberphase ist dann nämlich kaum Trägermaterial gelöst, sondern es befindet sich typischerweise mit einem Gehalt zwischen meist 25 und 40 Gew.-% an verdichtetem Gas in der schwereren Unterphase des Systems. Werden Glycerophospholipide als Trägermaterial verwendet, bildet sich unter Verwendung von Propangas bspw. bei Drücken zwischen 40 und 100 bar und Temperaturen zwischen 40 und 100°C eine Schmelze.

Optional können im vorliegenden Verfahren auch geringe Mengen eines geeigneten Schleppmittels, wie insbesondere kurzkettige C₁₋₄-Alkohole, Ester (z. B. Ethylacetat) oder Ketone (z. B. Aceton) in Konzentrationen bis zu 10 Gew.-% bezogen auf den zu vermahlenden Reaktorinhalt zugesetzt werden.

Es sei an dieser Stelle nochmals herausgestellt, daß die Zustandsbedingungen lediglich dazu führen, daß das Trägermaterial in der beschriebenen Art und Weise gelöst wird, die feste Verbindung jedoch im System zunächst schlecht bzw. nicht löslich ist. Die Voraussetzungen des erfindungsgemäßen Verfahrens mit erhöhtem Druck, erhöhter Temperatur und insbesondere die Möglichkeit einer hoch konzentrierten Zustandsform des Trägermaterials und hier insbesondere der oberflächenaktiven Verbindung in Form einer Schmelze, sind besonders geeignet, um den zu zermahlenden Wirkstoff in möglichst kleine Partikel bzw. durch den Mahlvorgang in Lösung zu überführen.

Entscheidend für den Erfolg des vorliegenden Verfahrens kann sein, daß nicht nur das Trägermaterial unter den jeweiligen Verfahrensbedingungen eine Schmelze bildet, sondern daß der gesamte Autoklaveninhalt durch den Mahlvorgang in eine homogene Schmelze überführt wird; diese Variante wird durch die Erfindung besonders berücksichtigt.

Empfohlen wird ebenfalls, daß im direkten Anschluß an den Verfahrensschritt a) und noch vor dem Verfahrensschritt b) die Rührerdrehzahl auf 50 bis 200 und vorzugsweise auf 100 U/min. reduziert wird. Mit dieser Maßnahme erhält das Homogenat die Möglichkeit, sich im Bodenbereich des Rührautoklaven zu sammeln ohne sich dabei ggf. entmischen zu können.

Nach dem eigentlichen Mahlvorgang unter Druck kann gemäß Erfindung anschließend im zweiten Verfahrensschritt b) das zermahlene Produkt vom verdichteten Gas befreit werden. Verfahrenstechnisch geschieht dies insbesondere beim Vorliegen einer homogenen Schmelze mit Hilfe einer Druckerniedrigung über eine Entspannungsdüse vorzugsweise gegen Atmosphärendruck, wobei dann ein pulverförmiges partikelreduziertes Formulierungsprodukt erhalten wird.

Zu beachten ist, daß in vielen Fällen der Entspannungsvorgang selbst insgesamt keinen wesentlichen Einfluß mehr auf die Partikelgröße der formulierenden Verbindung ausübt. Damit unterscheidet sich das erfindungsgemäße Verfahren deutlich vom Prinzip des RESS-Verfahrens, bei dem die Partikelgröße ausschließlich durch den Ausdüsvorgang des im überkritischen Gas gelösten Wirkstoffes determiniert wird.

Das erfindungsgemäße Verfahren kombiniert somit folgende physikalischen und physikalisch-chemischen Prinzipien zur Partikelreduzierung und gleichzeitigen Solubilisierung von schwer- oder nichtlöslichen festen Verbindungen (Wirkstoffen):

Bei erhöhtem Druck in einem verdichteten Gas, das ggf. selbst gewisse Löseeigenschaften für den Wirkstoff besitzt, und unter Einsatz von Trägermaterialien wird unter den Zustandsbedingungen des erfindungsgemäßen Verfahrens ein hochkonzentriertes Homogenat, vorzugsweise als Schmelze, erhalten, wobei die mechanische Zermahlung bevorzugt nach dem Prinzip einer Kugelmühle erfolgt und unter Umständen eine zusätzliche Reduzierung der Partikelgröße durch den Entspannungsvorgang aus dem verdichteten Gas möglich ist.

Die folgenden Beispiele sollen diese Vorteile des erfindungsgemäßen Verfahrens veranschaulichen.

### Beispiele

### Beispiel 1

20 g Aciclovir wurden mit 80 g ölfreiem Sojalecithin (nicht fraktioniert, bezogen von Lucas Meyer, Hamburg) trocken gemischt (Pulvermischung) und zusammen mit 70 ml Keramikkugeln (Durchmesser: 5 mm) in einen 400 ml-Rührautoklaven eingebracht. Der Rührautoklav wurde auf 60°C temperiert und mit Propangas auf 80 bar aufgedrückt, wobei das Lecithin in eine Schmelze überführt wurde. Anschließend wurde der Rührmotor eingeschaltet und der Mahlvorgang bei einer Rührergeschwindigkeit von 1000 U/min. unter Konstanthaltung von Druck und Temperatur über einen Zeitraum von 2 Stunden durchgeführt, wobei deutlich die Mahlgeräusche der Kugeln wahrgenommen werden konnten.

Nach diesen 2 Stunden wurde die Rührerdrehzahl auf ca. 100 U/min. reduziert, um der Schmelze die Gelegenheit zu geben, sich im Bodenbereich des Autoklaven zu sedimentieren. Anschließend wurde die so hergestellte Schmelze über ein Druckventil am Boden des Autoklaven von 80 bar auf Atmosphärendruck entspannt, wodurch eine spontane Verdampfung des Propangases erreicht und ein pulverförmiges Produkt erhalten wurde. Eine Gehaltsbestimmung ergab, daß das Aciclovir quantitativ in der Formulierung enthalten war.

In einer mikroskopischen Untersuchung wurde festgestellt, daß die definierten Teilchen des Ausgangsmaterials (Korngrößenspektrum zwischen 50 und 250 µm) aufgelöst und in die amorphe Matrix des Sojalecithins aufgenommen wurde. Eine mikroskopische Bestimmung der Partikelgröße des Aciclovirs war nicht mehr möglich.

### Beispiel 2

5 g kristallines β-Carotin (synthetisches Material, bezogen von BASF, Ludwigshafen) wurden mit 95 g ölfreiem lyso-Sojalecithin (Emulfluid®, bezogen von Lucas Meyer, Hamburg) trocken gemischt (Pulvermischung) und zusammen mit 70 ml Keramikkugeln (Durchmesser: 5 mm) in einen 400 ml-Rührautoklaven eingebracht. Der Rührautoklav wurde auf 80°C temperiert und mit einer Mischung bestehend aus 90 Gew.-% Propangas und 10 Gew.-% DME auf 100 bar aufgedrückt, wobei das lyso-Lecithin in eine Schmelze überführt wurde. Anschließend wurde der Mahlvorgang bei einer Rührergeschwindigkeit von 3000 U/min. und unter Konstanthaltung von Druck und Temperatur über einen Zeitraum von 0,5 Stunden durchgeführt.

Danach wurde die Rührerdrehzahl auf ca. 100 U/min. reduziert, um der Schmelze die Gelegenheit zu geben, sich im Bodenbereich des Autoklaven abzusetzen. Anschließend wurde die so hergestellte Schmelze über ein Druckventil am Boden des Autoklaven von 100 bar auf Atmosphärendruck entspannt, wodurch eine spontane Verdampfung des Gasgemisches erreicht und ein pulverförmiges, stark rot gefärbtes Produkt erhalten wurde.

In der mikroskopischen Untersuchung wurde festgestellt,daßdie kristallinen Strukturen des Ausgangsmaterials nahezu vollständig aufgelöst und in die amorphe Matrix des lyso-Sojalecithins eingearbeitet worden sind. In einer Analyse der Isomeren-Zusammensetzung wurde festgestellt, daß während des Formulierungsvorganges nur eine geringfügige Isomerisierung (< 5 %) des all-trans-β-Carotins stattgefunden hat.

### Beispiel 3

15 g β-Sitosterin wurden mit 85 g Alkylpolyglycosid (APG, Tego Care CG 90, bezogen von Th. Goldschmidt, Essen) trocken gemischt (Pulvermischung) und zusammen mit 70 ml Keramikkugeln (Durchmesser: 5 mm) in einen 400 ml-Rührautoklaven eingebracht. Der Rührautoklav wurde auf 120°C temperiert und mit einer Mischung bestehend aus 80 Gew.-% Propangas und 20 Gew.-% DME auf 80 bar aufgedrückt, wobei das APG in eine Schmelze überführt wurde. Anschließend wurde der Mahlvorgang bei einer Rührergeschwindigkeit von 1000 U/min. und unter Konstanthaltung von Druck und Temperatur über einen Zeitraum von 2,5 Stunden durchgeführt.

Danach wurde die Rührerdrehzahl auf ca. 100 U/min. reduziert, um der Schmelze die Gelegenheit zu geben, sich im Bodenbereich des Autoklaven abzusetzen. Anschließend wurde die so hergestellte Schmelze über ein Druckventil am Boden des Autoklaven von 80 bar auf Atmosphärendruck entspannt, wodurch eine spontane Verdampfung des Gasgemisches erreicht und ein pulverförmiges, homogen weißes Produkt erhalten wurde.

Die mikroskopische Untersuchung zeigte, daß die Partikelstrukturen des Ausgangsmaterials nahezu vollständig aufgelöst waren. Das Produkt zeigte eine stark verbesserte Dispergierbarkeit in lipophilen und hydrophilen Medien.

## Patentansprüche

1. Verfahren zur Herstellung pulverförmiger partikelreduzierter Formulierungen mit Hilfe verdichteter Gase, **dadurch gekennzeichnet, daß** man
a) die zu formulierende feste Verbindung in Gegenwart von verdichtetem Gas gemeinsam mit 10 bis 99 Gew.-% eines Trägermaterials bezogen auf das Gesamtgewicht der partikelreduzierten Formulierung aus Verbindung und Trägermaterial bei erhöhten Verfahrensdrücken homogen vermahlt, wobei das Trägermaterial im verdichteten Gas im Wesentlichen löslich ist, sowie
b) anschließend das verdichtete Gas durch Druckerniedrigung entspannt und vom Homogenat abtrennt sowie die pulverförmige partikelreduzierte Formulierung aus dem Homogenat gewinnt.

2. Verfahren nach Anspruch 1, **dadurch gekennzeichnet, daß** man zu formulierende feste Verbindungen einsetzt, die in Abwesenheit des Trägermaterials schwer- oder nichtlöslich im verdichteten Gas sind.

3. Verfahren nach Anspruch 1 oder 2, **dadurch gekennzeichnet, daß** man als zu formulierende feste Verbindung bioaktive Verbindungen, insbesondere pharmazeutische und/oder kosmetische Wirkstoffe und ganz besonders bevorzugt solche einsetzt, die zur peroralen, parenteralen, inhalativen, intravenösen, intramuskulären, subkutanen, intraperitonalen und/oder intranasalen sowie zur topischen oder transdermalen Applikation geeignet sind.

4. Verfahren nach Anspruch 1 oder 2, **dadurch gekennzeichnet, daß** man als zu formulierende feste Verbindung Agrochemikalien, wie Biozide und Pflanzenwachstumsregulatoren, einsetzt.

5. Verfahren nach Anspruch 1 oder 2, **dadurch gekennzeichnet, daß** man als zu formulierende feste Verbindung Baustoffchemikalien, wie Farblacke und Betonzusatzmittel, einsetzt.

6. Verfahren nach einem der Ansprüche 1 bis 5, **dadurch gekennzeichnet, daß** als Trägermaterial eine oberflächenaktive Substanz, besonders bevorzugt Phospholipide wie Glycerophospholipide, sowie Partialglyceride wie Mono- und Diglyceride, Kohlenhydrat(-derivate) wie Alkylpolyglycoside, Zuckerester und Sorbitanfettsäureester, sowie künstliche und natürliche Polymere, besonders bevorzugt Polyethylenoxid-Block-Copolymere ("Poloxamere"), Polyethylenglykole, Siliconderivate wie methyl- und/oder phenylsubstituierte Polysiloxane mit unterschiedlichem Vernetzungsgrad, und Gelatine(-derivate), und beliebige Mischungen daraus eingesetzt werden.

7. Verfahren nach einem der Ansprüche 1 bis 6, **dadurch gekennzeichnet, daß** im Verfahrensschritt a) 50 bis 90 Gew.-% bezogen auf die partikelreduzierte Formulierung an Trägermaterial zugegeben werden.

8. Verfahren nach einem der Ansprüche 1 bis 7, **dadurch gekennzeichnet, daß** als verdichtete Gase Propan und/oder Dimethylether und/oder Kohlendioxid sowie Mischungen aus Propan und/oder Dimethylether mit bis zu 90 Gew.-% bezogen auf die Gasmischung Butan oder Kohlendioxid, herangezogen werden.

9. Verfahren nach einem der Ansprüche 1 bis 8, **dadurch gekennzeichnet, daß** für das Vermahlen ein Rührautoklav mit integrierter Kugelmühle verwendet wird.

10. Verfahren nach einem der Ansprüche 1 bis 9, **dadurch gekennzeichnet, daß** im Verfahrensschritt a) das homogene Vermahlen bei einer Rührgeschwindigkeit von 500 bis 4000 U/min und insbesondere von 1000 bis 2000 U/min durchgeführt wird.

11. Verfahren nach einem der Ansprüchen 1 bis 10, **dadurch gekennzeichnet, daß** im Verfahrensschritt a) das homogene Vermahlen über einen Zeitraum von 0,5 bis 3 Stunden und insbesondere 1 bis 2 Stunden erfolgt.

12. Verfahren nach einem der Ansprüche 1 bis 11, **dadurch gekennzeichnet, daß** im Verfahrensschritt a) bis zu 10 Gew.-% bezogen auf den zu vermahlenden Inhalt eines Schleppmittels wie kurzkettige C₁₋₄-Alkohole, Ester (z. B. Ethylacetat) und Ketone (z. B. Aceton) zugesetzt werden.

13. Verfahren nach einem der Ansprüche 1 bis 12, **dadurch gekennzeichnet, daß** im Verfahrensschritt a) der Druck-auf 5 bis 500 bar, vorzugsweise auf 40 bis 120 bar eingestellt wird.

14. Verfahren nach einem der Ansprüche 1 bis 13, **dadurch gekennzeichnet, daß** im Verfahrensschritt a) die Temperatur auf 10 bis 200°C, vorzugsweise auf 50 bis 120°C eingestellt wird.

15. Verfahren nach einem der Ansprüche 1 bis 14, **dadurch gekennzeichnet, daß** im Verfahrensschritt a) der Autoklaveninhalt zu einer homogenen Schmelze vermahlen wird.

16. Verfahren nach einem der Ansprüche 1 bis 15, **dadurch gekennzeichnet, daß** am Ende des Verfahrensschritts a) die Rührerdrehzahl auf 50 bis 200 U/min und besonders bevorzugt auf 100 U/min reduziert wird.

17. Verfahren nach einem der Ansprüche 1 bis 16, **dadurch gekennzeichnet, daß** im Verfahrensschritt b) die Druckerniedrigung über eine Entspannungsdüse erfolgt.

## Claims

1. A process for the preparation of pulverulent particle-reduced formulations with the aid of compressed gases, **characterized in that**
a) the solid compound to be formulated is homogeneously ground at elevated process pressures in the presence of compressed gas together with 10 to 99% by weight of a carrier material based on the total weight of the particle-reduced formulation of compound and carrier material, the carrier material being essentially soluble in the compressed gas, and
b) the compressed gas is then expanded by lowering the pressure and separated off from the homogenate, and the pulverulent particle-reduced formulation is recovered from the homogenate.

2. The process as claimed in claim 1, **characterized in that** solid compounds to be formulated are employed which are poorly soluble or nonsoluble in the compressed gas in the absence of the carrier material.

3. The process as claimed in claim 1 or 2, **characterized in that**, as the solid compound to be formulated, bioactive compounds, in particular pharmaceutical and/or cosmetic active compounds, and very particularly preferably those compounds are employed which are suitable for peroral, parenteral, inhalational, intravenous, intramuscular, subcutaneous, intraperitoneal and/or intranasal administration and for topical or transdermal application.

4. The process as claimed in claim 1 or 2, **characterized in that**, as the solid compound to be formulated, agrochemicals, such as biocides and plant growth regulators, are employed.

5. The process as claimed in claim 1 or 2, **characterized in that**, as the solid compound to be formulated, construction chemicals, such as lacquers and concrete additives, are employed.

6. The process as claimed in one of claims 1 to 5, **characterized in that**, as the carrier material, a surface-active substance, particularly preferably phospholipids such as glycerophospholipids, and partial glycerides such as mono- and diglycerides, carbohydrate (derivatives) such as alkyl polyglycosides, sugar esters and sorbitan fatty acid esters, and also synthetic and natural polymers, particularly preferably polyethylene oxide block copolymers ("poloxamers"), polyethylene glycols, silicone derivatives such as methyl- and/or phenyl-substituted polysiloxanes having a differing degree of crosslinkage, and gelatin (derivatives), and any desired mixtures thereof are employed.

7. The process as claimed in one of claims 1 to 6, **characterized in that** in process step a) 50 to 90% by weight, based on the particle-reduced formulation, of carrier material are added.

8. The process as claimed in one of claims 1 to 7, **characterized in that**, as compressed gases, propane and/or dimethyl ether and/or carbon dioxide, and mixtures of propane and/or dimethyl ether with up to 90% by weight, based on the gas mixture, of butane or carbon dioxide are used.

9. The process as claimed in one of claims 1 to 8, **characterized in that**, for the grinding, a stirred autoclave having an integrated ball mill is used.

10. The process as claimed in one of claims 1 to 9, **characterized in that**, in process step a), the homogeneous grinding is carried out at a stirrer speed of 500 to 4000 rpm and in particular from 1000 to 2000 rpm.

11. The process as claimed in one of claims 1 to 10, **characterized in that**, in process step a), the homogeneous grinding is carried out over a period of 0.5 to 3 hours and in particular 1 to 2 hours.

12. The process as claimed in one of claims 1 to 11, **characterized in that**, in process step a), up to 10% by weight, based on the contents of an entraining agent to be ground, such as short-chain C₁₋₄-alcohols, esters (e.g. ethyl acetate) and ketones (e.g. acetone) are added.

13. The process as claimed in one of claims 1 to 12, **characterized in that**, in process step a), the pressure is adjusted to 5 to 500 bar, preferably to 40 to 120 bar.

14. The process as claimed in one of claims 1 to 13, **characterized in that**, in process step a), the temperature is adjusted to 10 to 200°C, preferably to 50 to 120°C.

15. The process as claimed in one of claims 1 to 14, **characterized in that**, in process step a), the contents of the autoclave are ground to give a homogeneous melt.

16. The process as claimed in one of claims 1 to 15, **characterized in that**, at the end of the process step a), the stirrer speed is reduced to 50 to 200 rpm and particularly preferably to 100 rpm.

17. The process as claimed in one of claims 1 to 16, **characterized in that**, in process step b), the lowering of the pressure is carried out by means of an expansion nozzle.

## Revendications

1. Procédé pour la préparation de formulations pulvérulentes à taux particulaire réduit à l'aide de gaz comprimés, **caractérisé en ce que** l'on
(a) broie de manière homogène le composé solide à formuler en présence de gaz comprimé ensemble avec 10 à 99 % en poids d'un matériau support par rapport au poids total de la formulation à taux particulaire réduit à partir du composé et du matériau support à des pressions de procédé élevées, le matériau support étant essentiellement soluble dans le gaz comprimé, et
(b) détend ensuite le gaz comprimé par diminution de pression et on le sépare de l'homogénat et on obtient la formulation pulvérulente à taux particulaire réduit à partir de l'homogénat.

2. Procédé selon la revendication 1, **caractérisé en ce que** l'on met en oeuvre des composés solides à formuler, qui sont difficilement ou non solubles dans le gaz comprimé en l'absence du matériau support.

3. Procédé selon la revendication 1 ou 2, **caractérisé en ce que** l'on met en oeuvre comme composé solide à formuler des composés bioactifs, en particulier des substances actives pharmaceutiques et/ou cosmétiques et de manière tout à fait préférée celles qui sont adaptées à l'application perorale, parentérale, par inhalation, par voie intraveineuse, intramusculaire, sous-cutanée, intrapéritonéale et/ou intranasale ainsi qu'à une application topique ou transdermique.

4. Procédé selon la revendication 1 ou 2, **caractérisé en ce que** l'on utilise comme composé solide à formuler des composés agrochimiques, comme des biocides et des régulateurs de croissance végétale.

5. Procédé selon la revendication 1 ou 2, **caractérisé en ce que** l'on met en oeuvre comme composé solide à formuler des produits chimiques pour la construction, comme des peintures et des additifs du béton.

6. Procédé selon l'une des revendications 1 à 5, **caractérisé en ce que** l'on utilise comme matériau support une substance tensioactive, de manière particulièrement préférée des phospholipides comme des glycérophospholipides, et des glycérides partiels comme des mono- et diglycérides, des (dérivés de) glucides comme des alkylpolyglycosides, des esters de sucre et des esters d'acides gras avec le sorbitane, ainsi que des polymères naturels et synthétiques, de manière particulièrement préférée des copolymères séquencés de polyoxyde d'éthylène (« poloxamères »), des polyéthylène-glycols, des dérivés du silicium comme des polysiloxanes méthyl- et/ou phényl-substitués avec différents degrés de réticulation, et des (dérivés de) gélatine, et des mélanges quelconques de ceux-ci.

7. Procédé selon l'une des revendications 1 à 6, **caractérisé en ce que** l'on ajoute à l'étape de procédé a) 50 à 90 % en poids de matériau support par rapport à la formulation à taux particulaire réduit.

8. Procédé selon l'une des revendications 1 à 7, **caractérisé en ce que** l'on a recours comme gaz comprimés à du propane et/ou du diméthyléther et/ou du dioxyde de carbone ainsi qu'à des mélanges de propane et/ou de diméthyléther avec jusqu'à 90 % de butane ou de dioxyde de carbone en poids par rapport au mélange de gaz.

9. Procédé selon l'une des revendications 1 à 8, **caractérisé en ce que** l'on utilise pour le broyage un autoclave agité avec un broyeur à billes intégré.

10. Procédé selon l'une des revendications 1 à 9, **caractérisé en ce que** l'on réalise à l'étape de procédé a) le broyage homogène à une vitesse d'agitation de 500 à 4000 tr/min et en particulier de 1000 à 2000 tr/min.

11. Procédé selon l'une des revendications 1 à 10, **caractérisé en ce que** l'on réalise à l'étape de procédé a) le broyage homogène sur une période de 0,5 à 3 heures et en particulier 1 à 2 heures.

12. Procédé selon l'une des revendications 1 à 11, **caractérisé en ce que** l'on ajoute à l'étape de procédé a) jusqu'à 10 % en poids d'un agent entraînant comme des alcools à chaîne courte en C₁ à C₄, des esters (par exemple de l'acétate d'éthyle) et des cétones (par exemple de l'acétone) par rapport à la quantité à broyer.

13. Procédé selon l'une des revendications 1 à 12, **caractérisé en ce que** l'on ajuste à l'étape de procédé a) la pression à 5 à 500 bars, de préférence 40 à 120 bars.

14. Procédé selon l'une des revendications 1 à 13, **caractérisé en ce que** l'on ajuste à l'étape de procédé a) la température à 10 à 200 °C, de préférence 50 à 120 °C.

15. Procédé selon l'une des revendications 1 à 14, **caractérisé en ce que** l'on broie à l'étape de procédé a) le contenu de l'autoclave en une fonte homogène.

16. Procédé selon l'une des revendications 1 à 15, **caractérisé en ce que**, à la fin de l'étape de procédé a), on réduit la vitesse d'agitation à 50 à 200 tr/min et de manière particulièrement préférée à 100 tr/min.

17. Procédé selon l'une des revendications 1 à 16, **caractérisé en ce que**, à l'étape de procédé b), on effectue la diminution de pression au moyen d'une buse de détente.
